# EUROPEAN PATENT SPECIFICATION

(11) **EP 3 061 488 B1**
(45) Date of publication and mention of the grant of the patent: **20.10.2021**
(21) Application number: 15197286.6
(22) Date of filing: 25.11.2014
(51) Int. Cl.: A61M 25/10, A61L 29/06, A61B 17/3207, A61L 29/14, A61L 29/18, A61B 17/22

(54) **MEDICAL BALLOON**
MEDIZINISCHER BALLON
BALLONNET MEDICAL

(30) Priority: 29.11.2013 GB 201321063; 07.11.2014 GB 201419864
(43) Date of publication of application: 31.08.2016
(62) Divisional of application: 14275246.8
(73) Proprietor: Cook Medical Technologies LLC, Bloomington, IN 47404 (US)
(72) Inventor: Aggerholm, Steen, 4660 St. Heddinge (DK); Lysgaard, Thomas, 2680 Solroed Strand (DK)
(74) Representative: Williams Powell

(56) References cited:
- US-A1- 2003 144 683
- US-A1- 2005 038 383
- US-A1- 2009 234 283
- US-A1- 2011 160 756
- US-B1- 6 875 197
- US-B1- 6 905 743
- None

## Description

### Technical Field

The present invention relates to a medical balloon.

### Background Art

Traditionally, vessel dilatation was effected by open surgery procedures but advances in medical technology have enabled such procedures to be carried out endoluminally, principally by means of expandable medical devices of which angioplasty balloons have proven most effective. Some angioplasty balloons have scoring or cutting elements attached to the outside of the main body portion of the balloon, the cutting elements for instance being in the form of sharp metal blades. Another example provides scoring or cutting elements which are integrally formed with the balloon. The scoring or cutting elements are provided to break up plaque within the vessel and thus recanalise the latter. The balloon acts to keep the blades or elements pressed against the vessel wall during the scoring or cutting process and also acts to dilate the vessel as the process is carried out.

A problem with balloons formed with separate scoring or cutting blades fixed to the balloon is that the manufacturing process is more complex, time consuming and costly. Moreover, the eventual structure generally requires a fixation element to fix the blade to the balloon, which is typically in the form of a base support and adhesives or other bonding agents. The resultant structure can be relatively bulky, leading to limitations in the ability to fold and wrap the balloon to small diameters for delivery purposes. This can result in the balloon being unsuitable for smaller vessels or for the treatment of highly restricted vessels. Moreover, the balloon structure can be relatively rigid, leading to loss of flexibility of the balloon when deflated and as a result reduced trackability through tortuous vasculature.

A cutting or scoring balloon formed with integral cutting or scoring elements can resolve many of the shortcomings of balloons formed with separate scoring or cutting elements. However, in some instances the cutting or scoring elements and/or the overall structure of the balloon can be less rigid when deployed, leading to a reduction in cutting or scoring efficiency.

There is also a risk during angioplasty procedures that the balloon may tear, primarily as a result of the existence or generation of sharp edges of plaque and/or the pressure to which the balloon is inflated to during the process. While a tear which propagates in a longitudinal direction of the balloon tends not to be critical, as the balloon can be removed easily form the patient in one piece and thus replaced, tears which propagate circumferentially can be problematic, particularly if this leads to portions of the balloon snagging within the vessel or breaking off.

Furthermore, scoring or cutting balloons tend to be difficult to detect during imaging as a result of their inherent imaging transparency.

At least some of the problems identified above can be exhibited in balloons used for other than angioplasty procedures.

Some examples of cutting or scoring balloons can be found in US-2003/0163148, US-2012/0130407, US-2009/0234283, US-2011/0160756, US-2004/0230178 and US-2003/0153870.

Other existing documents include US 6,875,197, US2003/144683, US2009/234283, US 6,905,743, US2005/038383, US2011/160756.

### Disclosure of the Invention

The present invention seeks to provide an improved medical balloon and is defined by the appended claims.

According to an aspect of the present invention, there is provided a medical balloon including a balloon body member made of a first polymer material; the body member being disposed between first and second balloon ends and having a balloon wall with a wall thickness and inner and outer wall surfaces, the outer wall surface being generally rounded in a circumferential direction of the body member; and at least one elongate element on the balloon wall extending between the first and second ends of the balloon body member, the at least one elongate element being made of a second polymer material having a greater rupture strength compared to a rupture strength of the first polymer material, and wherein the at least one elongate element has a flattened outer surface, the at least one elongate element has an outer surface which is substantially smooth with the outer wall surface of the balloon body member. Most preferably, the at least one elongate element has an outer surface substantially flush with the outer wall surface of the balloon body member.

The preferred example provides a balloon which effects no scoring or cutting function but which has increased resistance against tear propagation and which can therefore be operated at higher pressures.

The at least one elongate element may have a base extending into the balloon wall, which may extend into the balloon wall by at least 50 percent of the thickness of the balloon wall; for substantially the entire thickness of the balloon wall; or to the inner wall surface of the balloon wall.

The second polymer material may have a greater rigidity than a rigidity of the first polymer material.

The first and second polymer materials may be of the same polymer type.

The first and second polymer materials may include the same polymer.

One of the first and second polymer materials may include one of more of: an additive, a structural modification and a blend, modifying its characteristics relative to the other of the first and second polymer materials.

The second material may be amorphous.

The first and second materials may include Nylon, preferably Nylon 12.

The at least one elongate element and the balloon body member may be co-extruded.

The second polymer material may for example be or include radiopaque or echogenic material.

The balloon may include a plurality of said elongate elements, said elements being spaced from one another circumferentially around the balloon body member.

Other features of the apparatus disclosed herein will become apparent from the following specific description of preferred examples.

### Brief Description of the Drawings

Examples of the present invention are described below, by way of example only, with reference to the accompanying drawings, in which:
Figure 1 is a schematic diagram of an example of medical balloon;
Figure 2 is a transverse cross-sectional view of an example of raw tubing for forming the balloon of Figure 1;
Figures 3 and 4 are sketches of transverse sections of two different manufactured raw tubing according to the teachings herein;
Figure 5 is a transverse cross-sectional view of another example of raw tubing for forming the balloon of Figure 1;
Figure 6 is sketches of a transverse section of a manufactured raw tubing according to Figure 4;
Figure 7 is a transverse cross-sectional view of another example of raw tubing for forming a medical balloon as taught herein;
Figures 8A to 8D are sketches of different examples of raw tubing and blown balloons according to the teachings herein.

### Description of the preferred examples

There are described below various examples of medical balloon which can be used in a variety of medical procedures. A number of the examples are to a balloon which is provided with a plurality of scoring or cutting elements extending along the outer surface of the balloon and which can be used for removing debris and in particular plaque from within a vessel, as well as for vessel dilatation. Other examples are directed to a balloon having strengthening elements or strips extending along the length of the balloon and which do not perform any scoring or cutting action. The strengthening elements are designed to allow an increase in the rate of inflating pressure of a balloon by reducing or eliminating the risk of circumferential tear propagation. The concepts taught herein can be used for a variety of medical balloons.

The person skilled in the art will appreciate that the drawings are not to scale and often depict various parts of the balloon in significantly enlarged form. Moreover, the proportions of the various elements of the balloon are not as they would be in practice. The schematic form of the drawings is intended to show clearly the different parts of the structure. A person skilled in the art will immediately appreciate, from common general knowledge, the typical dimensions and relative proportions of the various elements of the balloon, and also that these will also vary in dependence upon the specific medical application.

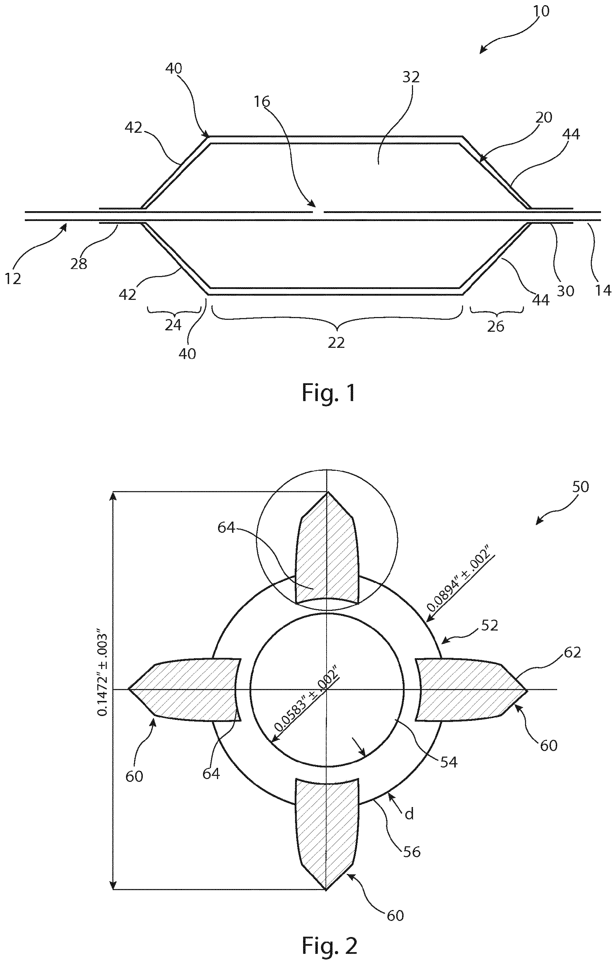

Referring now to Figure 1, this shows in schematic form and in side elevation an example of medical balloon 10 shown in cross-section. The balloon 10 is carried on a balloon catheter 12 of conventional form, which terminates at a distal end 14. The balloon catheter 12 includes one or more apertures 16 in its wall, communicating with the catheter lumen, for providing inflation fluid into the balloon 20, as well as for exhausting fluid from the balloon 20 in order to deflate this for removal purposes.

In the example shown in Figure 1 the balloon 20 has a generally conventional form with, in this example, a body element 22 of substantially cylindrical shape and which is of elongate form, that is has a length greater than its diameter. A person skilled in the art will appreciate that medical balloons may have a variety of different shapes and they may sometimes be wider than they are longer.

The balloon 20 also includes end portions 24, 26, which are typically conical as shown in Figure 1. Beyond the end cones 24, 26, the balloon 20 has neck portions 28, 30 which are fixed to the balloon catheter 12 in fluid tight manner, so that the balloon 20 has at least one chamber 32 which is sealed, save for the fluid connection through the aperture 16 of the balloon catheter 12.

The example of balloon 10 shown in Figure 1 is a scoring or cutting balloon and in this regard is provided with a plurality of elongated scoring or cutting elements 40, which preferably extend for the whole length of the balloon 20 and most preferably also along the length of the end cones 24, 26 and of the necks 28, 30. Having scoring elements 40 of this nature can provide longitudinal strength to the balloon 20 and, as explained below, can provide increased tear resistance, specifically by providing a mechanism for inhibiting or substantially reducing the risk of circumferential tear propagation.

The scoring elements 40 may extend longitudinally along the balloon 20, that is in a direction parallel to the longitudinal axis of the balloon 20, which could be equated with the longitudinal axis of the balloon catheter 12 as viewed in Figure 1. It is not excluded, however, that the scoring elements 40 could extend at an angle to this and may, for example, run helically along and around the balloon 20. The actual disposition of the scoring or cutting elements 40 on the balloon, as well as the number of scoring elements 40 provided on the balloon, will typically vary dependent on the medical procedure and the vessel size in which the balloon is to be used. This will be within the understanding and knowledge of the person of average skill in the art.

In Figure 1, the scoring or cutting elements 40 as shown as having a substantially uniform height along the body portion 22 of the balloon 20, although they may have a varying height in some examples, for example to have discontinuities or teeth along the lengths of the scoring elements 40, useful in the breaking up of plaque within a vessel. The portions 42, 44 of the scoring elements which extend along the end cones 24, 26 of the balloon 20 preferably have heights substantially less than their height along the main body portion 22 of the balloon 20. In examples where the scoring elements 40 extend all the way into the neck portions 28, 30 also, the scoring elements preferably have a reduced height at the necks also and in practice preferably as little as possible. It is likewise preferred that the portions of the scoring elements 40 extending along the end cones is also minimised. Given that the end cones 24, 26 and the necks 28, 30 of the scoring elements 40 will generally provide no substantial scoring or cutting function, is not necessary for them to protrude from the balloon wall at all or by any significant amount and it is also not necessary for them to have pointed ends or edges. It is preferred, in fact, that the portions of the scoring elements 40 which extend along the ends cones 24, 26 (that is the portions 42, 44) and along the necks 28, 30 be as thin as possible as this enhances the flexibility of the balloon 20, particularly when the balloon is in a deflated and folded condition, which it would be during endoluminal delivery of the balloon to the treatment site in the patient.

As will be apparent from the description and drawings which follow, in examples having scoring or cutting elements 40, these preferably have an outermost point or apex for use in scraping or cutting plaque from within a vessel wall. It is not excluded, though, that the scoring elements could have a rounded extremity. In all the preferred examples, the scoring elements will extend by an operative height beyond the outer surface of the balloon wall so as to present a discontinuity in the outer surface of the balloon. As explained below, the scoring elements 40 are also preferably made of a more rigid material than that of the balloon wall.

Although Figure 1 shows only two scoring elements 40, it is to be understood that there will typically be provided more than two scoring elements on the balloon 20, for example four, six or more, or any number therebetween. The actual number can be selected by the person skilled in the art, typically in dependence upon the condition and type of vessel to be treated. It is also feasible that in some examples, in addition to scoring elements extending longitudinally along the balloon 20, there may be provided scoring elements which extend at an angle thereto. For the reasons indicated below, though, it is preferred that the scoring elements 40 extend along the length of the balloon and substantially parallel to the longitudinal axis of the balloon 20.

It will be appreciated that the scoring elements 40 will be spaced circumferentially around the balloon 20, and in particular the body portion of the balloon 20. They are preferably equally spaced around the balloon 20.

In the preferred examples described with reference to Figures 2-8 below, the balloon wall is depicted as being a single layer of material. This is, however, not essential as the balloon wall could be formed of a plurality of layers of balloon wall material. These layers may have different characteristics and have different functions.

Figures 2 to 7 show various examples of raw balloon tubing. The balloon raw tubing is a precursor to the medical balloon and is typically inflated in a mold at high temperature to form the medical balloon. The mold will have internal walls with a shape equivalent to the desired final shape of the balloon. Thus, the mold may have a shape equivalent to the balloon shape shown in the example of Figure 1, or any other balloon shape desired to be formed.

Figures 2 to 4 show an example of raw balloon 50 with ribs 60 which will form the scoring elements or strengthening elements of the balloon. More specifically, referring first to Figure 2, this shows in transverse cross-section an example of raw balloon form 50 which has a tubing portion 52 which will form the balloon wall once the raw tubing is inflated in a suitable mold. The tubing portion 52 has an inner surface 54, which forms the inner surface of the balloon, and an outer surface 56 which will form the outer surface of the balloon. The raw tubing has a wall thickness d, which will reduce as the raw tube 50 is inflated in the mold, as the skilled person will be fully aware. The tubing portion 52 could be made of a single layer of material, as in the example shown, or could be made of a plurality of layers of the same material or materials having different constituents and characteristics.

The raw tubing 50 also includes, in this example, four ribs 60, which extend lineally along the tubing and are equally spaced circumferentially around the tubing portion 52, so as to be equidistant from one another. Each rib 60 includes an apex or pointed extremity 62, which may be uniform along the entire length of the ribs 60, but which in other examples may be non-uniform so as to give the ribs 60 a non-smooth, for instance a castellated or toothed, form. The ribs 60 each have a base 64 which extends into the thickness of the tubing portion 52, as will be apparent in particular from Figure 2. As a result of this disposition of the ribs 60, the outer surface 56 of the tubing portion 52 is discontinuous around the circumference of the tubing portion 52. In other words, the outer surface of the balloon raw tubing 50 comprises portions of tubing 52 and the ribs 60.

The base 64 of each rib 60 preferably extends a significant amount into the thickness d of the tubing portion 52, preferably at least 50% or more of this thickness. It is preferred also that the base 64 of each rib 60 is curved in the circumferential direction, as depicted in Figure 2, with a radius of curvature which approaches the radius curvature of the tubing portion 52 at which the bases 64 are located. Specifically, in the example shown in Figure 2, the radii of the bases 64 are equivalent to the radius of the tubing portion at the depth of the bases 64. In other examples described below, where the ribs 60 extend all the way to the inner wall 54, the radii of curvature of the bases 64 will be the same or substantially the same as the radius curvature of the inner surface 54 of the tubing portion 52. This assists in ensuring smooth expansion of the balloon raw tubing 50 during the formation of the eventual balloon. It is, however, not essential that the ribs 60 have the shape shown in Figure 2, in particular their bases 64 may have different shapes.

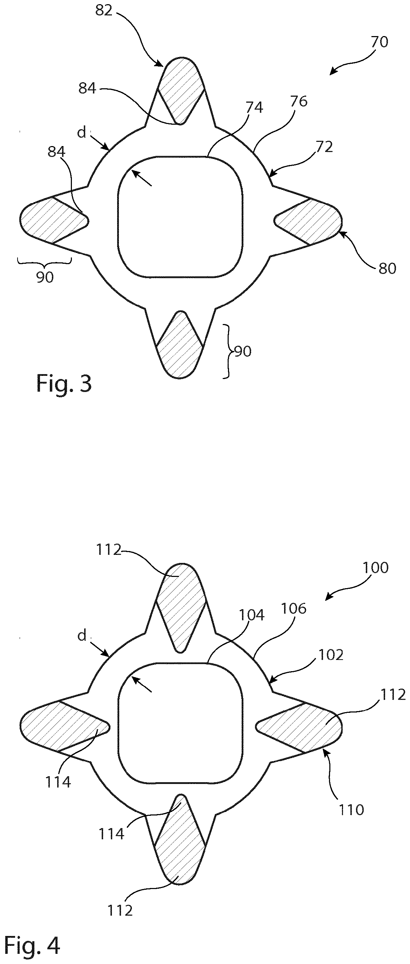

Referring now to Figures 3 and 4, these show two examples of manufactured raw tubing in accordance with the teachings herein. The raw tubing shown in Figures 3 and 4 are transverse cross-sectional views of an actual extruded tubing. Referring first to Figure 3, the balloon raw tubing 70 is provided with a tubing portion 72 having an inner wall surface 74 and an outer wall surface 76. The raw tubing 70 also includes, as with the example of Figure 2, four ribs 80 which extend longitudinally along the raw tubing and are equidistantly spaced from one another in the circumferential direction. The ribs 80 include apices 82 at their extremities and also extend into the depth of the tubing portion 72. In the example of Figure 3, it can be seen that during extrusion the tubing portion 72 will flow into the zone or depth 90 of each rib 80. In the example of Figure 3, the ribs 80 are such that once the raw tubing is inflated to form the balloon, the ribs, which will form the scoring or cutting elements, will extend into the thickness or depth of the balloon wall, formed by the tubing portion 72. This will occur as the ribs will tend to spread during inflation much less than the tubing portion 72 and they will thus thin less.

The bases 84 of the ribs 80 are, in this example, pointed compared to the bases 64 of the example of Figure 2.

With reference now to Figure 4, the example of balloon raw tubing 100, being similar to the raw tubing 70 of Figure 3 and 50 of Figure 2, includes a tubing portion 102 having inner and outer wall surfaces 104, 106 and ribs 110 which extend a substantial way into the thickness or depth d of the tubing portion 102 and in this embodiment very close to the inner wall surface 104 of the tubing portion 102. Again, as with the example of Figure 3, the ribs 110 have apices 112 and bases 114 which are pointed. The ribs 110, which will form the scoring or cutting elements of the balloon, will extend a substantial way into the depth of the balloon wall.

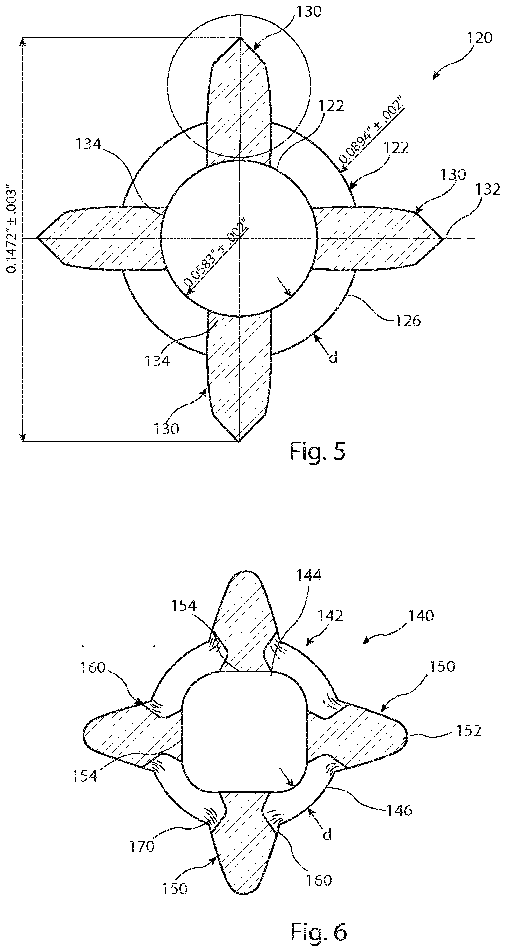

Referring now to Figures 5 and 6, these show another example of balloon raw tubing 120, 140 having similar characteristics to those already described. In the example of Figure 5, the raw tubing 120 includes a tubing portion 122 having an inner wall surface 124 and an outer wall surface 126 with a thickness or depth d. Spaced equidistantly in a circumferential direction are four ribs 130, which extend longitudinally along the tubing portion 122. Each rib has an apex or point 132 and a base 134 which in this example extends to the inner wall surface 124 of the tubing portion 52. Thus, the ribs 130 extend through the whole thickness or depth d of the tubing portion 122.

The example of balloon raw tubing 140 shown in Figure 6 has equivalent characteristics to the balloon raw tubing 120 of the example in Figure 5 and is a cross-sectional view of a raw tubing. The raw tubing 140 includes a tubing portion 142 having an inner wall surface 144, an outer wall surface 146 and a thickness or depth d. The ribs 150 are similar to the ribs of the previously described examples and comprise apices 152 extending along their length and have bases 154 which extend to the inner wall surface 144 and in other words for the whole of the thickness or depth d of the tubing portion 142. Figure 6 also shows that part of the tubing portion 142 will, during the extrusion process, extend into the zone of the ribs 150 and in particular at the junction 160 with the ribs 150. The tubing portion 52 may therefore be somewhat thicker at this junction 160, as with the examples of Figures 3 and 4.

Figure 6 also shows zones 170, by the junction 160 between the tubing portion 142 and the ribs 150, in which the materials or constituents of the tubing portion 142 and the ribs 150 will to at least a certain extent mix together during the extrusion process. This can be useful in providing a gradual transition in changes in material of the balloon raw tubing 140, which can enhance the strength of the raw tubing and eventual balloon. This occurs particularly when the ribs extend into the depth of the tubing portion, which enables an amalgamation or mixing of the materials of these elements of the balloon raw tubing during the extrusion process.

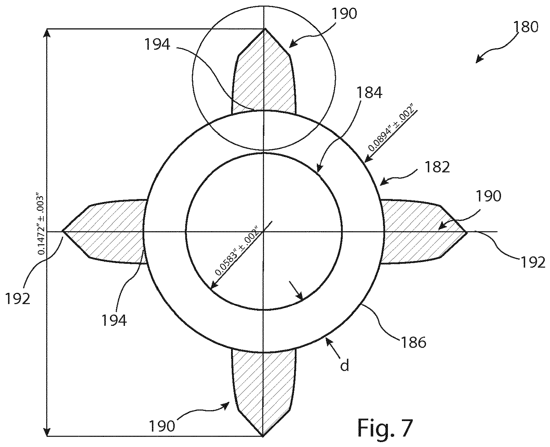

Figure 7 shows an another example of balloon raw tubing 180, which is similarly provided with a tubing portion 182 having an inner wall surface 184 and an outer wall surface 186, with a wall depth or thickness d. The raw tubing 180 also includes, in this example, four ribs 190 extending in the longitudinal direction of the tubing portion 182 and which are provided with apices 192 at their extremities, which are pointed as in all of the previous examples, and bases 194 which in this example extend to the position of the outer wall surface 186 of the tubing portion 182. In other words, the ribs 190 do not extend, in this example, into the wall of the tubing portion 182. This example is used particularly for the production of a medical balloon having radiopaque properties and/or a medical balloon having strengthening elements.

As explained above, even though the examples of Figures 2 to 7 show four ribs spaced equidistantly in the circumferential direction of the tubing portion, a different number of ribs may be provided in other examples (whether fewer or more). The ribs need not extend precisely parallel to the longitudinal axis of the tubing portion but may, as previously explained, have different configurations, for example helical or any of the other configurations described herein.

The ribs of the examples of raw tubing described in connection with Figures 2 to 7 and similarly in all of the examples described herein are preferably made of materials having different characteristics to the material or materials of which the tubing portions are made. In one example, for instance, the ribs are made of a material which has a greater rigidity than the rigidity of the material or materials forming the tubing portion. In another example, the material of the ribs has a greater rupture or tear strength than the rupture or tear strength of the material or materials forming the tubing portion. In yet another example, the ribs may be formed of or include radiopaque and/or echogenic material, whereas the tubing portion is formed of a material which is substantially radio transparent. The ribs may have any one of or any combination of these characteristics and in the preferred example are made of material with greater rupture strength, which is radiopaque and which optionally also has greater rigidity.

It is preferred that the ribs and tubing portions are made from or include polymer materials and in particular polymer materials which are compatible with one another. In particular, the ribs and tubing portions are preferably made of polymer materials which are able to blend and mix with one another without any noticeable interface or transition point once formed, particularly by extrusion. For this purpose, the ribs and tubing portions may be made of the same polymer type or even of the same polymer. Preferably the ribs are made of an amorphous polymer material, preferably an amorphous polyamide material, and the tubing portions are made of a polymer material of the same type or even including the same polymer but which is not amorphous.

In one example one or the other or both of the materials of the ribs and tubing portions is provided with an additive, a structural modification and/or a blend which modifies its characteristics relative to the other material. For instance, the material forming the ribs may have an additive which increases the rigidity, rupture strength, radiopacity and/or echogenicity of the material forming the ribs in comparison with the material or materials of which the tubing portion is made. In other examples, the ribs may be formed of a material which is structurally modified compared to that of the tubing portions, for example by a greater degree of cross-linking or other modification which will be apparent to the person skilled in the art.

In one example, the tubing portion is made of non-amorphous Nylon 12, and the ribs are made from amorphous Nylon 12. One example of amorphous Nylon 12 is Grilamid TR55.

Amorphous polymers, in particular amorphous Nylon 12 have been found to be particularly rigid and easily shaped; they are more rigid that their non-amorphous counterparts and can easily be shaped by a mould into the desired rib configuration.

In another example, the tubing portion is made of a polyamide such as Nylon 12 and the ribs are made of a different or different form of polyamide such as Nylon 66 or Nylon 6. Nylon 66 and Nylon 6 have a greater rupture strength and are more rigid than Nylon 12. Being of the same polymer type the tubing portions and ribs will co-extrude as a unitary material without any weakened transition point between two materials. It is also preferred, as described above, that the ribs are formed of a mixture or blend with a radiopaque and/or echogenic material.

In all of the examples described and contemplated herein, the radiopaque and/or echogenic material or additive may be one or more of: tungsten, gold, platinum, palladium, barium or bismuth. Barium and bismuth are radiopaque; whereas tungsten, gold, platinum and palladium are both radiopaque and echogenic. Echogenic materials include PVC and fluoropolymers. These materials thus can provide good radiopacity, and/or echogenicity, and are biocompatible. Tungsten is the most preferred material as this has very good performance even when used in small amounts. Materials which are solely echogenic can be seen by fluoroscopy techniques.

It is preferred that the ribs include between 50 and 90% by weight of radiopaque/echogenic material, more preferably, between 60 and 80% by weight of radiopaque and/or echogenic material. A layer with 80% of tungsten has been found to be particularly effective.

In terms of concentration by volume, the radiopaque/echogenic material may comprise substantially 11.4% to substantially 20.6%, more preferably substantially 13.7% to substantially 18.3%, most preferably 14.8% to 18.3% by volume.

It will be apparent that the ribs will preferably be formed form a mix or blend of radiopaque/echogenic material and polymeric material.

It is also envisaged that the tubing portion may be made of a blend of polymers, as can the ribs. It is preferred that the polymeric materials of the first and second materials are the same or of the same type and co-extruded. This ensures a strong and unitary coupling of the elements to one another, and in some instances at least a seamless interface between the elements. In another example, the polymeric materials of the tubing portion and the ribs are different.

Although some examples use Nylon as the core constituent of the balloon and ribs or scoring/cutting elements, the polymeric material of the ribs and tubing portions may include one or more of polyamide, polyether block amide (Pebax), PET, polyethylene and polyurethane.

The ribs may have any of the characteristics mentioned herein.

The raw tubing disclosed herein and exemplified by the examples of Figures 2 to 7 are used to form a medical balloon, as described, by placing the raw tubing in a suitable mold and then inflating this while heating so that the tubing expands to form a balloon shape of a type similar to the medical balloon of Figure 1. In so doing, the tubing portions of the raw tubing will expand and stretch, with the thickness or depth thereof reducing and eventually being the thickness of the balloon wall. The ribs may also be stretched but typically to a far lesser extent than the tubing portion, typically as a result of the greater rigidity or strength of the material forming the ribs. The base of the ribs will typically remain within the same relative position in the balloon wall as with the raw tubing and thus, in the preferred examples, be at least partially embedded within the depth of the formed balloon wall. The ribs can be made to retain their pointed extremities by providing suitable grooves within the mold surface to receive the ribs and ensure that they are not flattened during the blowing and heating process. As explained above, it is preferred that the ribs are flattened at the end cones of the balloon and also the necks of the balloon, the latter typically being formed of non-inflated raw tubing. Flattening can be produced within the mold, for instance by ensuring that the mold surfaces which form the conical end cones of the balloon and the ends of the mold which withhold the neck portions of the balloon, have smooth internal surfaces which will act to flatten the ribs during the balloon blowing process. In other examples the ribs could be reduced in height/depth subsequently to blowing the balloon, for instance by cutting, abrading or etching these. An example includes laser cutting. As described above, reducing the height of the ribs at the conical portions of the balloon and at its necks will increase the flexibility of the balloon, particularly when the balloon is deflated. and will hence increase the trackability of the balloon for endoluminal delivery purposes.

In some examples, as described below, the ribs may be flattened along the entirety of the length of the balloon.

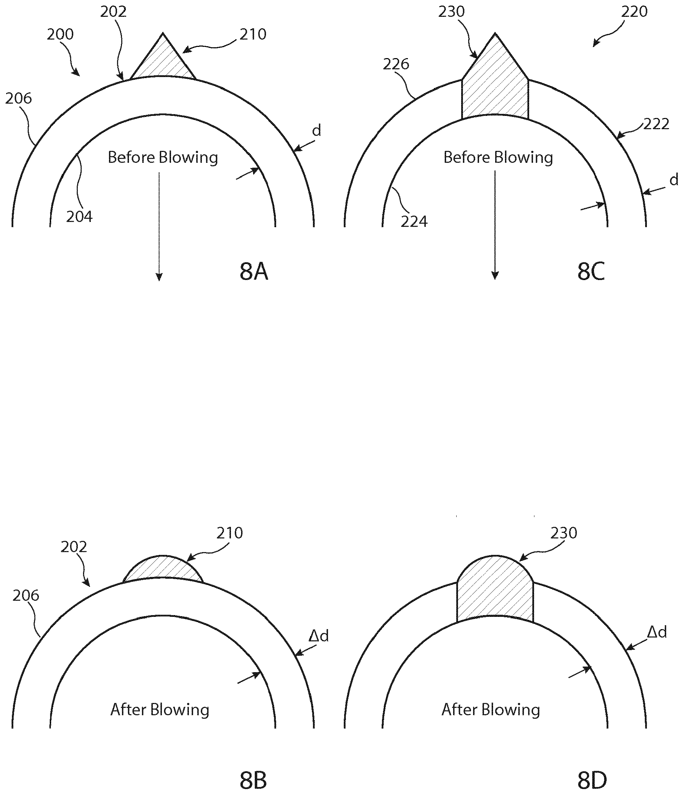

Other examples of raw tubing and blown balloon are shown in Figure 8.

These are just two examples from the large variety of examples contemplated herein, as will be apparent from the disclosure as a whole.

Figures 8A and 8B show an example having similar characteristics to the example of Figure 7, in which a raw tubing 200 has a tubing portion 202 having an inner wall surface 204 and an outer wall surface 206. Attached to the outer wall surface 206 and formed preferably by co-extrusion are a plurality of ribs 210 which extend longitudinally along the raw tubing and preferably parallel to the longitudinal axis of the raw tubing. The ribs 210 (of which there may be four, fewer or more) are preferably made of a material which has a greater rupture strength and/or is radiopaque or echogenic. As will be apparent from a comparison of Figures 8A and 8B, when the raw tubing 200 is blown, that is inflated while being heated in a mold, the ribs 210 will flatten (and the tubing portion 202 will stretch circumferentially and reduce in thickness to a thickness Δ *d*, in known manner). The ribs 210 preferably provide a flattened outer surface which is most preferably substantially smooth with the outer wall surface 206 of the balloon 202 and most preferably substantially flush with the outer wall surface. The reader will appreciate that Figure 8B shows the ribs 210 with a height which is exaggerated compared to the preferred examples described and this is solely for the sake of clarity of what is depicted in the drawings.

Figures 8C and 8D show another example in which the raw tubing 220, formed of a tubing portion 222 which forms the balloon wall, has inner and outer surfaces 224, 226 and a plurality of ribs 230 which include a base extending all the way to the inner wall surface 224. After blowing in a mold and forming the balloon, the ribs 230 are flattened in the same way as the ribs 210 of the examples of Figures 8A and 8B and as described herein.

The various examples of raw tubing and balloon disclosed herein provide a medical balloon with various advantages over the art. For instance, where the balloon is provided with cutting and scoring elements which extend into the thickness of the balloon wall, these can provide resistance to or prevention of circumferential propagation of any tears to the balloon during its use. Specifically, should the balloon tear during use, for example as the result of plaque in a vessel wall or the application of too much pressure to the balloon, any such tear would tend to propagate along the balloon wall. Such a tear could propagate along the length of the balloon where there is no scoring or cutting element or other strengthening element arranged cross-wise around the balloon but this would still ensure the balloon fragments remain attached to the balloon catheter 12. On the other hand, any tears which propagate circumferentially around the balloon would eventually come up against the cutting or scoring element and be unable to propagate beyond this. The cutting or scoring element will therefore stop circumferential propagation of the tear.

Moreover, having cutting or scoring elements extending into the thickness of the balloon wall provides greater support to the cutting or scoring elements and in particular enables these to apply more cutting or scoring pressure to plaque within the vessel wall. Cutting or scoring elements which extend all the way to the inner wall surface of the balloon wall are considered to be particularly effective.

Increased resistance to circumferential tear propagation can also be achieved with the provision of flattened strengthening elements extending along the length of the balloon, whether or not such elements act as cutting or scoring elements for an angioplasty procedure. In such examples, as described herein, the flattening strengthening elements could be disposed on the outside of the balloon wall and equally could extend into the depth of the balloon wall, either for the entirety of the thickness of the balloon wall or to be embedded only partially within its thickness.

The provision of radiopaque material in the cutting/scoring or strengthening elements enables the balloon to be seen during the medical procedure by standard imaging techniques. It is particularly advantageous to have the radiopaque elements formed within a polymer material which is the same as or the same type, or compatible in terms of blending, as the material from which the balloon wall is made, which enables the raw tubing to be produced in a single coextrusion. This is a simpler process and can provide a balloon having better structural integrity than a balloon made in separate stages with components which are separately attached to one another.

In examples which have the ribs or scoring/cutting elements extending to the inner wall surface of the balloon, that is which separate the balloon wall circumferentially through its complete thickness, there may be provided a thin internal layer to the structure, which is provided specifically to assist in the raw tube blowing process. The thin internal layer does not provide any significant structural change to the formed balloon. Such a layer may be made of the same material as the balloon wall but could equally be made of a different material such as: Pebax, Nylon 12, PET or similar material.

## Claims

1. A medical balloon including a balloon body member made of a first polymer material; the body member being disposed between first and second balloon ends and having a balloon wall (182, 202) with a wall thickness and inner and outer wall surfaces, the outer wall surface being generally rounded in a circumferential direction of the body member; and at least one elongate element (190, 210) on the balloon wall (182, 202) extending between the first and second ends of the balloon body member, the at least one elongate element (190, 210) being made of a second polymer material having a greater rupture strength compared to a rupture strength of the first polymer material, and wherein the at least one elongate element (190, 210) has a flattened outer surface; wherein the at least one elongate element (190, 210) has an outer surface which is substantially smooth with the outer wall surface of the balloon body member.

2. A medical balloon according to claim 1, wherein the at least one elongate element (190, 210) has an outer surface substantially flush with the outer wall surface of the balloon body member.

3. A medical balloon according to any preceding claim, wherein the second material includes an amorphous polymer material.

4. A medical balloon according to any preceding claim, wherein the second polymer material has a greater rigidity than a rigidity of the first polymer material.

5. A medical balloon according to any preceding claim, wherein the first and second polymer materials are of the same polymer type.

6. A medical balloon according to any preceding claim, wherein the first and second polymer materials include the same polymer.

7. A medical balloon according to any preceding claim, wherein one of the first and second polymer materials include one of more of: an additive, a structural modification and a blend, modifying its characteristics relative to the other of the first and second polymer materials.

8. A medical balloon according to any preceding claim, wherein the second material includes Nylon, preferably Nylon 12.

9. A medical balloon according to any preceding claim, wherein the at least one elongate element (190, 210) and the balloon body member are co-extruded.

10. A medical balloon according to any preceding claim, wherein the balloon includes a plurality of said elongate elements (190, 210), said elements (190, 210) being spaced from one another circumferentially around the balloon body member.

## Patentansprüche

1. Medizinischer Ballon, der ein Ballonkorpuselement, das aus einem ersten Polymermaterial gefertigt ist; wobei das Korpuselement zwischen ersten und zweiten Ballonenden angeordnet ist und eine Ballonwand (182, 202) mit einer Wanddicke sowie Innen- und Außenwandflächen aufweist, wobei die Außenwandfläche allgemein in einer Umfangrichtung des Korpuselements abgerundet ist; und mindestens ein längliches Element (190, 210) auf der Ballonwand (182, 202) einschließt, das sich zwischen den ersten und zweiten Enden des Ballonkorpuselements erstreckt, wobei das mindestens eine längliche Element (190, 210) aus einem zweiten Polymermaterial mit einer größeren Bruchfestigkeit gefertigt ist, verglichen mit einer Bruchfestigkeit des ersten Polymermaterials, und wobei das mindestens eine längliche Element (190, 210) eine abgeflachte Außenfläche aufweist; wobei das mindestens eine längliche Element (190, 210) eine Außenfläche aufweist, die im Wesentlichen nahtlos mit der Außenwandfläche des Ballonkorpuselements ist.

2. Medizinischer Ballon nach Anspruch 1, wobei das mindestens eine längliche Element (190, 210) eine Außenfläche hat, die im Wesentlichen bündig mit der Außenwandfläche des Ballonkorpuselements ist.

3. Medizinischer Ballon nach einem der vorhergehenden Ansprüche, wobei das zweite Material ein amorphes Polymermaterial einschließt.

4. Medizinischer Ballon nach einem der vorhergehenden Ansprüche, wobei das zweite Polymermaterial eine größere Steifheit als eine Steifheit des ersten Polymermaterials hat.

5. Medizinischer Ballon nach einem der vorhergehenden Ansprüche, wobei das erste und das zweite Polymermaterial von demselben Polymertyp sind.

6. Medizinischer Ballon nach einem der vorhergehenden Ansprüche, wobei das erste und das zweite Polymermaterial das gleiche Polymer einschließen.

7. Medizinischer Ballon nach einem der vorhergehenden Ansprüche, wobei eines von dem ersten und dem zweiten Polymermaterial ein oder mehrere von einem Additiv, einer Strukturmodifikation und einem Gemisch einschließt, wodurch dessen Charakteristika relativ zu dem anderen von dem ersten und dem zweiten Polymermaterial modifiziert werden.

8. Medizinischer Ballon nach einem der vorhergehenden Ansprüche, wobei das zweite Material Nylon einschließt, vorzugsweise Nylon 12.

9. Medizinischer Ballon nach einem der vorhergehenden Ansprüche, wobei das mindestens eine längliche Element (190, 210) und das Ballonkorpuselement coextrudiert sind.

10. Medizinischer Ballon nach einem der vorhergehenden Ansprüche, wobei der Ballon eine Vielzahl der länglichen Elemente (190, 210) einschließt, wobei die Elemente (190, 210) im Umfang voneinander um das Ballonkorpuselement herum beabstandet sind.

## Revendications

1. Ballonnet médical comprenant un élément de corps de ballonnet constitué d'un premier matériau polymère ; l'élément de corps étant disposé entre des première et seconde extrémités de ballonnet et comportant une paroi (182, 202) de ballonnet ayant une épaisseur de paroi et des surfaces interne et externe de paroi, la surface de paroi externe étant généralement arrondie dans une direction circonférentielle de l'élément de corps ; et au moins un élément allongé (190, 210) sur la paroi de ballonnet (182, 202) s'étendant entre les première et seconde extrémités de l'élément de corps de ballonnet, l'au moins un élément allongé (190, 210) étant constitué d'un second matériau polymère ayant une résistance à la rupture plus grande comparativement à une résistance à la rupture du premier matériau polymère, et dans lequel l'au moins un élément allongé (190, 210) possède une surface externe aplatie ; dans lequel l'au moins un élément allongé (190, 210) possède une surface externe qui est sensiblement uniforme avec la surface de paroi externe de l'élément de corps de ballonnet.

2. Ballonnet médical selon la revendication 1, dans lequel l'au moins un élément allongé (190, 210) possède une surface externe sensiblement de niveau avec la surface de paroi externe de l'élément de corps de ballonnet.

3. Ballonnet médical selon l'une quelconque des revendications précédentes, dans lequel le second matériau comprend un matériau polymère amorphe.

4. Ballonnet médical selon l'une quelconque des revendications précédentes, dans lequel le second matériau polymère présente une rigidité plus grande qu'une rigidité du premier matériau polymère.

5. Ballonnet médical selon l'une quelconque des revendications précédentes, dans lequel les premier et second matériaux polymères sont du même type de polymère.

6. Ballonnet médical selon l'une quelconque des revendications précédentes, dans lequel les premier et second matériaux polymères comprennent le même polymère.

7. Ballonnet médical selon l'une quelconque des revendications précédentes, dans lequel un parmi les premier et second matériaux polymères comprend un ou plusieurs parmi : un additif, une modification structurale et un mélange, modifiant ses caractéristiques par rapport à l'autre parmi les premier et second matériaux polymères.

8. Ballonnet médical selon l'une quelconque des revendications précédentes, dans lequel le second matériau comprend du Nylon, de préférence du Nylon 12.

9. Ballonnet médical selon l'une quelconque des revendications précédentes, dans lequel l'au moins un élément allongé (190, 210) et l'élément de corps de ballonnet sont coextrudés.

10. Ballonnet médical selon l'une quelconque des revendications précédentes, dans lequel le ballonnet comprend une pluralité desdits éléments allongés (190, 210), lesdits éléments (190, 210) étant espacés l'un de l'autre circonférentiellement autour de l'élément de corps de ballonnet.
